(19) Europäisches Patentamt
European Patent Office
Office européen des brevets

(11) Numéro de publication: **0 226 501 B1**

# (12) FASCICULE DE BREVET EUROPEEN

(45) Date de publication du fascicule du brevet: **28.02.90**

(51) Int. Cl.⁴: **C07C 231/02**, C07C 233/38, C07D 233/36

(21) Numéro de dépôt: **86402575.4**

(22) Date de dépôt: **20.11.86**

(54) **Nouveau procédé de synthèse de (méth)acrylamide de N-dialkylaminoalkyle.**

(30) Priorité: **27.11.85 FR 8517506**

(43) Date de publication de la demande: **24.06.87 Bulletin 87/26**

(45) Mention de la délivrance du brevet: **28.02.90 Bulletin 90/9**

(84) Etats contractants désignés: **AT BE CH DE ES FR GB GR IT LI LU NL SE**

(56) Documents cités:
**EP-A- 0 117 530**
**FR-A- 2 246 538**
**GB-A- 2 021 101**

(73) Titulaire: **NORSOLOR S.A., Tour Aurore Place des Reflets, F-92080 Paris la Défense Cédex 5(FR)**

(72) Inventeur: **Hurtel, Patrice, 2, Chemin des Brassens, F-57500 Saint-Avold(FR)**
Inventeur: **Laurent, Denis, 31, Parc du Tyrol, F-57500 Saint-Avold(FR)**

(74) Mandataire: **Rieux, Michel, c/o NORSOLOR Service Propriété Industrielle B.P. 57, F-62670 Mazingarbe(FR)**

EP 0 226 501 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un nouveau procédé de synthèse de (méth)acrylamide de N-dialkylaminoalkyle, c'est-à-dire des méthacrylamides de N-dialkylaminoalkyle et des acrylamides de N-dialkylaminoalkyle.

On entend par (méth)acrylamide de N-dialkylaminoalkyle, les composés de formule générale :

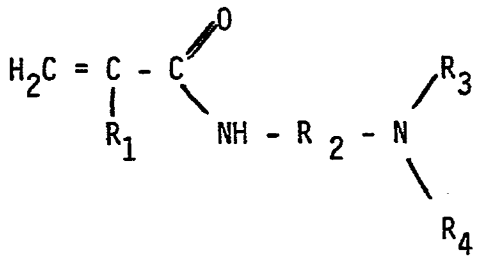

dans laquelle :

-$R_1$ est un radical méthyle ou un atome d'hydrogène,

-$R_2$ est un groupe alkyle, linéaire ou ramifié, comprenant au plus 10 atomes de carbone,

-$R_3$ et $R_4$, identiques ou différents l'un de l'autre, peuvent être :

. soit deux groupes alkyles aliphatiques de 1 à 4 atomes de carbone,

. ou, $R_3$ est un groupe alkyle aliphatique de 1 à 4 atomes de carbone et $R_4$ est un groupe cycloaliphatique de 5 à 6 atomes de carbone,

.ou, $R_3$ et $R_4$ sont deux groupes cycloalphatiques de 5 à 6 atomes de carbone,

.ou, $R_3$ et $R_4$ forment avec l'atome d'azote, avec lequel ils sont liés chimiquement, un groupe hétérocycloaliphatique pouvant être notamment la pipéridine, la pyrrolidine.

Plusiers voies de synthèse de ces composés sont actuellement bien connues.

On connait notamment les réactions qui font intervenir un acide méthacrylique, ou ses esters, et une amine. Ce type de réaction présente le grave inconvénient d'être concurrencé par une réaction secondaire : il s'agit de la réaction de Michaël, c'est-à-dire l'addition de l'amine sur la double liaison (méth)acrylique. Pour cette raison, les différents procédés de synthèse de (méth)acrylamide de N-dialkylaminoalkyle sont des procédés complexes à plusieurs étapes de réaction.

Ainsi, le procédé décrit dans le brevet U.S. 3 878 247 et le brevet Canadien 1 020 163, consiste à faire réagir une diamine de formule $(R_2)(R_3)N - (CH_2)_n - NH_2$ (où $R_2$ et $R_3$ sont notamment des groupes alkyles) avec un acide ou un ester de formule $H_2C = CR_1 - COOZ$ (où $R_1$ est un radical méthyle ou un atome d'hydrogène, et Z est un atome d'hydrogène ou un groupe alkyle). Cette réaction donne un produit intermédiaire, résultat de l'amidification de la fonction acide ou de la fonction ester du dérivé (méth)acrylique, et de l'addition de Michaël de la diamine sur la double liaison méthacrylique. Il s'agit de la β-aminopropionamide de formule :

$(R_2)(R_3)N - (CH_2)_n - NH - CH_2 - CH(R_1) - CO - NH - (CH_2)_n - N(R_2)(R_3)$ La décomposition thermique de ce produit intermédiaire à 210-250 °C, en présence d'un catalyseur (U.S. 3 878 247), ou sans catalyseur mais à 180-300 °C (CA. 1 020 163), donne le (méth)acrylamide de N-dialkylaminoalkyle.

On connait également des procédés de synthèse de (méth)acrylamide de N-dialkylaminoalkyle dans lesquels la réaction de Michaël est rendue impossible.

Selon une première solution, décrite notamment dans le brevet allemend 2 856 383 et dans le brevet européen 13416, on fait réagir un amide d'acide carboxylique-β-hydroxylé avec une amine, et ensuite, on déshydrate le produit intermédiaire à une température comprise entre 200 et 400 °C, pour obtenir finalement le (méth)acrylamide souhaité.

Une autre solution consiste à utiliser un catalyseur qui inhibe la réaction de Michaël de l'amine sur la double liaison de l'ester (méth)acrylique. Les catalyseurs préconisés dans ce type de réaction sont notamment les organostanneux dans le brevet U.S. 4 321 411, les oxydes dialkylés d'étain dans le brevet allemand 2 816 516, ou encore le titanate d'alkyle dans le brevet allemand 3 048 020. L'utilisation de cette solution exige des précautions pour ne pas empoisonner le catalyseur.

La présente invention, qui a comme objectif de résoudre les problèmes des procédés connus, propose un nouveau procédé, simple dans sa mise en oeuvre, et qui permet d'obtenir des (méth)acrylamides substitués sans traces de produits secondaires, avec des rendements élevés.

Plus précisement, la présente invention a pour objet un nouveau procédé de synthèse de (méth)acrylamide de N-dialkylaminoalkyle de formule :

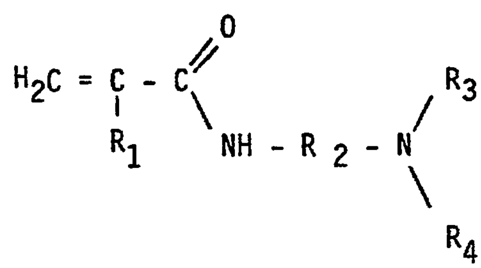

dans laquelle :

- $R_1$ est un radical méthyle ou un atome d'hydrogène,

- R $_2$ est un groupe alkyle, linéaire ou ramifié comprenant au plus 10 atomes de carbones,

- $R_3$ et $R_4$, identiques ou différents, peuvent être :

.soit deux groupes alkyles aliphatiques de 1 à 4 atomes de carbone,

.ou, $R_3$ est un groupe alkyle aliphatique de 1 à 4 atomes de carbone et $R_4$ est un groupe cycloaliphatique de 5 à 6 atomes de carbone,

.ou $R_3$ et $R_4$ sont deux groupes cycloaliphatiques de 5 à 6 atomes de carbone,

.ou, $R_3$ et $R_4$ forment avec l'atome d'azote, avec lequel ils sont liés chimiquement, un groupe hétérocycloaliphatique pouvant être la pipéridine, la pyrrolidine,

procédé caractérisé en ce qu'on fait réagir un anhydride (méth)acrylique de formule :

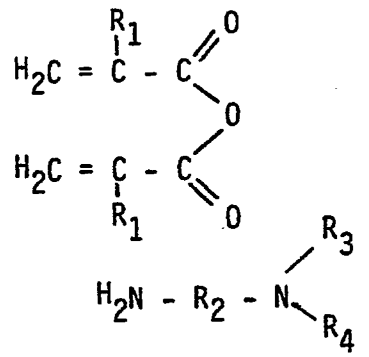

en présence d'au moins un inhibiteur de polymérisation, à une température comprise entre 70 et 100°C et en présence d'oxygène, puis on sépare le (méth)acrylamide de N-dialkylaminoalkyle formé.

De manière suprenante, le procédé selon l'invention permet d'atteindre des rendements élevés en (méth)acrylamide de N-dialkylaminoalkyle, supérieurs à 80%, avec des temps de réaction courts compris généralement entre 15 et 30 minutes.

En effet, la présence d'oxygène dans le milieu réactionnel permet d'inhiber la polymérisation des composés insaturés présents, à savoir le (méth)acrylamide de N-dialkylaminoalkyle et l'anhydride (méth)acrylique, bien que la température de réaction soit élevée. De plus, le produit final obtenu présente une bonne stabilité au stockage. L'oxygène utilisé dans le procédé selon l'invention peut par exemple être apporté en effectuant la réaction en présence d'air ou encore en ménageant un courant d'oxygène dans le milieu réactionnel.

Parmi les inhibiteurs de polymérisation utilisables dans le procédé selon l'invention, on peut citer l'hydroquinone, l'étherméthylique d'hydroquinone, la phénothiazine, le 2,6-tertbutyl-p-crésol, ou le bleu de méthylène ou des sels de cuivre comme le sulfate de cuivre, l'acétate de cuivre ou le chlorure cuivrique, dans un rapport pondéral par rapport à l'anhydride (méth)acrylique supérieur ou égal à 0,25%.

Conformément à un mode de réalisation, la séparation du (méth)acrylamide de N-dialkylaminoalkyle est effectuée par neutralisation de l'acide (méth)acrylique formé à l'aide d'une base forte, par exemple de la soude, et ensuite par extraction du (méth)acrylamide de N-dialkylaminoalkyle avec un solvant organique. Selon l'invention, on utilise de préférence des solvants benzéniques,par exemple du benzène, ou du toluène.

Selon un autre mode de réalisation préféré de l'invention, le (méth)acrylamide de N-dialkylaminoalkyle est séparé par distillation, cette opération étant effectuée en présence d'oxygène. Dans ce cas, on a trouvé que la séparation du (méth)acrylamide de N-dialkylaminoalkyle est parfaite, si on ajoute dans le milieu réactionnel, avant et/ou après la réaction entre l'anhydride de (méth)acrylique et la diamine, un solvant inerte vis-à-vis du milieu réactionnel, formant avec ce dernier un milieu homogène et possédant une température d'ébullition comprise entre celle de l'acide (méth)acrylique et celle du (méth)acrylamide de N-dialkylaminoalkyle. En effet, le milieu réactionnel obtenu après réaction qui comprend essentiellement l'acide (méth)acrylique et le (méth)acrylamide de N-dialkylaminoalkyle est visqueux. Par distillation, on ne parvient pas à séparer correctement les produits de la réaction, et on constate la présence d'impuretés provenant de la polymérisation de ces produits. Or, il a été trouvé que la présence d'un solvant interte et de température d'ébullition intermédiaire de celle de l'acide (méth)acrylique et de celle du (méth)acrylamide de N-dialkylaminoalkyle permet de séparer parfaitement par distillation les produits de la réaction et d'obtenir finalement du (méth)acrylamide de N-dialkylamino- alkyle pur.Ce solvant est ajouté dans le milieu réactionnel à raison de 10 à 50 % en poids par rapport à la charge totale des réactifs, et de préférence, à raison de 20 à 30 % en poids par rapport à la charge totale des réactifs.

Parmi les solvants utilisables dans le procédé selon l'invention, on peut citer le trichloro-1,2,4-benzène et ses isomères, le dichloro-1,2-benzène et ses isomères, ou des esters, par exemple l'acétate de phényle, l'acétate d'octyle, l'acétate de terpenyle ou l'acétate de tetrahydrofurfuryle, dans la mesure où la température d'ébullition de ces solvants possède un point d'ébullition intermédiaire entre ceux de l'acide et de l'amide formés lors de la réaction.

Les anhydrides concernés par le procédé selon l'invention sont notamment l'anhydride méthacrylique et l'anhydride acrylique.

Parmi les diamines de formule

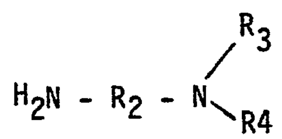

utilisables dans le procédé selon l'invention, on peut citer notamment :
la N,N-diméthylaminoéthylènediamine, la N,N-diméthylaminotriméthyllène-1,3-diamine, la N,N-diméthylaminotétraméthylène-1,4-diamine, la N,N,2,2-tétraméthylpropane-1,3-diamine, la pipéridine-N-alkylamine ou la pyrrolidine-N-alkylamine, l'aminoéthylimidazolidone, etc...

De préférence, le rapport molaire entre l'anhydride (méth)acrylique et la diamine est choisi entre 1 et 1,2.

De préférence encore, la réaction selon l'invention est réalisée à pression atmosphérique.

Le procédé selon l'invention est avantageux à plus d'un titre.

Le procédé selon l'invention ne comporte qu'une seule étape réactionnelle, ce qui facilite son exploitation industrielle.

De plus, comme indiqué auparavant, le procédé selon l'invention permet d'atteindre des rendements élevés de (méth)acrylamide de N-dialkylaminoalkyle, le plus souvent supérieur à 80%.

Les exemples donnés ci-dessous, à titre indicatif et non limitatif, permettront de mieux comprendre l'invention. Tous les essais décrits dans ces exemples sont effectués à la pression atmosphérique, donc

en présence d'air. La séparation du produit obtenu par distillation est effectuée à pression réduite, un bullage d'oxygène étant ménagé dans le milieu réactionnel.

Exemple 1 : Préparation du Méthacrylamide de diméthylaminopropyle

Dans un réacteur muni d'un dispositif réfrigérant et d'un système d'agitation mécanique, on introduit la charge suivante :
154 g d'anhydride méthacrylique,
0,75 g de bleu de méthylène,
0,75 g d'acétate de cuivre.

Quand la température de mélange réactionnel atteint 75 °C à la pression atmosphérique, on coule dans ce mélange :
102 g de N,N-diméthylaminopropylamine,
77 g de trichloro-1,2,4-benzène.

Après réaction, qui s'effectue quasi instantanément par la mise en contact des réactifs, on ajoute 0,75 g de phénothiazine et 0,75 g de 2,6-terbutyl-p-crésol et le mélange réactionnel est distillé sous vide. On distille en tête l'acide méthacrylique qui s'est formé lors de la réaction. L'acide méthacrylique passe à 88 °C sous une pression de 40 mmHg (53.3 mbar). On recueille 86 g d'acide méthacrylique. On distille ensuite le trichloro-1,2,4-benzène à 65°C sous une pression de 4 mmHg (5.3 mbar. Puis on récupère le méthacrylamide de diméthylaminopropyle à 124°C sous 4 mmHg (5.3 mbar). On recueille 156,7 g de méthacrylamide de diméthylaminopropyle.

Le rendement est de 88 %.

Exemple 2 : Préparation de l'acrylamide de diméthylaminonéopentyle

Dans un appareillage identique à celui décrit dans l'exemple 1, on introduit dans le réacteur la charge suivante :
189 g d'anhydride acrylique,
195 g de N,N,2,2-tétraméthylapropane-1,3-diamine,
115 g de trichloro-1,2,4-benzène,
0,75 g de bleu de méthylène,
0,75 g de sulfate de cuivre.

La réaction est menée sous agitation, à une température de 75°C et à la pression atmosphérique. Après réaction, qui s'effectue quasi instantanément par la mise en contact des réactifs, on ajoute dans le milieu réactionnel :
0,75 g de phénothiazine,
0,75 g de N,N'-phényl-paraphénylenèdiamine,
0,75 g d'éther méthylique d'hydroquinone.

On procède alors à la séparation par distillation des produits de la réaction. On distille en tête l'acide acrylique qui passe à 81°C sous 100 mmHg (133 mbar). On recueille 108 g d'acide acrylique. Ensuite, on distille le trichloro-1,2,4-benzène qui passe à 65°C sous 4 mmHg (5.3 mbar). Puis on récupère le diméthylaminonéopentylacrylamide à 131°C sous 8 mmHg (10.6 mbar). On recueille 226 g de diméthylaminonéopentylacrylamide.

Le rendement est de 82 %.

Example 3 : Préparation de l'acrylamide de diméthylaminopropyle

Dans un appareillge identique à celui décrit dans l'exemple 1, on introduit dans le réacteur la charge suivante :
204 g de N,N-diméthylaminopropylamine,
137 g de trichloro-1,2,4-benzène,
3000 ppm de bleu de méthylène.

On verse ensuite 252 g d'anhydride acrylique dans le réacteur, la température dans le réacteur étant de 80°C. Après réaction, qui s'effectue quasi instantanément par la mise en contact des réactifs, on ajoute 1500 ppm d'éthermethylique d'hydroquinone et 1500 ppm de phénothiazine et le mélange réactionnel est distillé sous vide. On distille en tête l'acide acrylique qui s'est formé lors de la réaction. L'acide acrylique passe à 81°C sous une pression de 100 mmHg (133 mbar). On recueille 144 g d'acide acrylique. On distille ensuite le trichloro-1,2,4-benzène à 78°C sous une pression de 5 mmHg (6.6 mbar). Puis on récupère l'acrylamide de diméthylaminopropyle à 129°C sous 3mmHg (4 mbar). On recueille 270,8 g d'acrylamide de diméthylaminopropyle.

Le rendement est de 90%.

Exemple 4 : Préparation du méthacrylamidoéthylimidazolidone

Dans un réacteur muni d'un dispositif réfrigérant et d'un système d'agitation mécanique, on introduit la charge suivante :
485,1 g d'anhydride méthacrylique,
500 g de trichloro-1-2-4-benzène,
500 ppm de phénothiazine,
1 000 ppm d'éthermethylique de l'hydroquinone.

Le milieu réactionnel ainsi constitué est agité vigoureusement ; on ajoute ensuite 387 g d'aminoéthylimidazolidone.

Pendant toute la durée de la réaction, le milieu réactionnel est maintenu à la pression atmosphérique et la température est maintenue à 75°C. L'acide méthacrylique formé lors de la réaction est distillé, en présence d'air. Celui-ci passe à 82°C sous une pression de 30 mm Hg (40 mbar). Le mélange trichlorobenzène et méthacrylamidoimidazolidone est ensuite refroidi. Les cristaux de méthacrylamidoimidazolidone précipitent, puis sont ensuite filtrés, lavés à l'acétone, puis séchés.

On recueille ainsi 450 g de monomère recherché.

Point de fusion : 114°C.

Rendement : 76%.

## Revendications

1) Procédé de synthèse de (méth)acrylamide de N-dialkylaminoalkyle de formule:

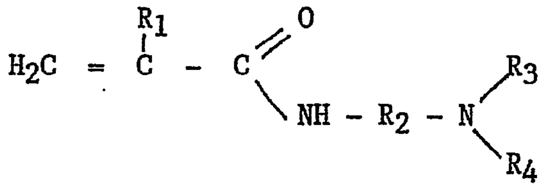

dans laquelle :

– $R_1$ est un radical méthyle ou un atome d'hydrogène,

– $R_2$ est un groupe alkyle, linéaire ou ramifié, comprenant au plus 10 atomes de carbone,

– $R_3$ et $R_4$, identiques ou différents, peuvent être:

. soit deux groupes alkyles aliphatiques de 1 à 4 atomes de carbone

. ou $R_3$ est un groupe alkyle aliphatique de 1 à 4 atomes de carbone et $R_4$ est un groupe cycloaliphatique de 5 à 6 atomes de carbone,

. ou $R_3$ et $R_4$ sont deux groupes cycloaliphatiques de 5 à 6 atomes de carbone,

. ou $R_3$ et $R_4$ forment avec l'atome d'azote, avec lequel ils sont liés chimiquement, un groupe hétérocycloaliphatique,

par réaction à partir d'un anhydride (méth)acrylique de formule:

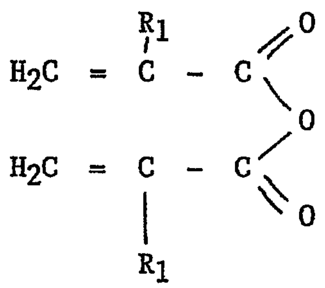

et en présence d'au moins un inhibiteur de polymérisation, caractérisé en ce qu'on fait réagir ledit anhydride avec une diamine de formule:

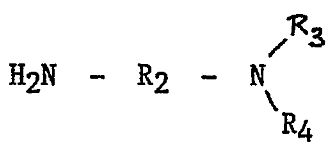

à une température comprise entre 70 et 100°C et en présence d'oxygène.

2) Procédé selon la revendication 1, caractérisé en ce que le (méth)acrylamide de N-dialkylaminoalkyle obtenu est séparé par distillation, et en ce que alors on ajoute dans le milieu réactionnel, avant et/ou après la réaction entre l'anhydride (méth)acrylique et la diamine, un solvant inerte avec le milieu réactionnel, formant avec ce dernier un milieu homogène et possédant une température d'ébullition comprise entre celle de l'acide (méth)acrylique et celle du (méth)acrylamide de N-dialkylaminoalkyle.

3) Procédé selon la revendication 2, caractérisé en ce que la quantité de solvant introduite dans le milieu réactionnel est comprise entre 10 et 50% en poids par rapport à la charge totale des réactifs.

4) Procédé selon la revendication 3, caractérisé en ce que la quantité introduite dans le milieu réactionnel est comprise entre 20 et 30 % en poids par rapport à la charge totale des réactifs.

5) Procédé selon l'une quelconque des revendications 1 à 4, caractérisé en ce que le solvant inerte est le trichloro-1,2,4-benzène ou l'un de ses isomères.

6) Procédé selon la revendication 1 caractérisé en ce que le (méth)acrylamide de N-dialkylaminoalkyle obtenu est séparé par extraction avec un solvant organique, après neutralisation du milieu réactionnel avec une base forte.

7) Procédé selon l'une quelconque des revendications 1 à 6, caractérisé en ce que le rapport molaire de l'anhydride (méth)acrylique sur la diamine est compris entre 1 et 1,2.

8) Procédé selon l'une quelconque des revendications 1 à 7, caractérisé en ce que la quantité d'inhibiteur de polymérisation est telle que le rapport pondéral de cet inhibiteur sur l'anhydride (méth)acrylique est supérieur ou égal à 0,25%.

## Claims

1. Process for the synthesis of an N-dialkylaminoalkyl(meth)acrylamide of formula:

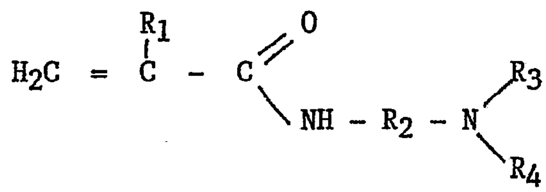

in which:

– $R_1$ is a methyl radical or a hydrogen atom,

– $R_2$ is a straight-chain or branched alkyl group containing at most 10 carbon atoms,

– $R_3$ and $R_4$, which are identical or different, may be:

either two aliphatic alkyl groups containing from 1 to 4 carbon atoms,

or $R_3$ is an aliphatic alkyl group containing from 1 to 4 carbon atoms and $R_4$ is an alicyclic group containing from 5 to 6 carbon atoms,

or $R_3$ and $R_4$ are two cycloaliphatic groups containing from 5 to 6 carbon atoms,

or $R_3$ and $R_4$ form, together with the nitrogen atom to which they are chemically linked, a heterocycloaliphatic group,

by reaction of a (meth)acrylic anhydride of formula:

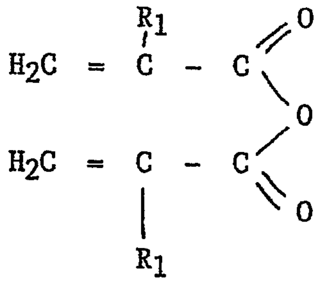

and in that presence of at least one polymerization inhibitor, characterized in that the said anhydride is reacted with a diamine of formula:

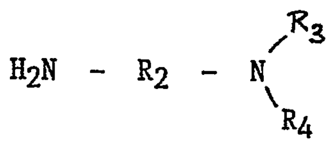

at a temperature of between 70 and 100°C and in the presence of oxygen.

2. Process according to Claim 1, characterized in that the N-dialkylaminoalkyl(meth)acrylamide obtained is separated by distillation and in that, before and/or after the reaction between (meth)acrylic anhydride and the diamine, there is added to the reaction mixture a solvent which is inert to the reaction mixture, which forms a homogeneous mixture with the latter, and which has a boiling temperature lying between that of (meth)acrylic acid and that of the N-dialkylaminoalkyl(meth)acrylamide.

3. Process according to Claim 2, characterized in that the quantity of solvent which is introduced into the reaction mixture is between 10 and 50% by weight based on the total charge of the reactants.

4. Process according to Claim 3, characterized in that the quantity introduced into the reaction mixture is between 20 and 30% by weight based on the total charge of the reactants.

5. Process according to any one of Claims 2 to 4, characterized in that the inert solvent is 1,2,4-trichlorobenzene or one of its isomers.

6. Process according to Claim 1, characterized in that the N-dialkylaminoalkyl(meth)acrylamide obtained is separated by extraction with an organic solvent after neutralization of the reaction mixture using a strong base.

7. Process according to any one of Claims 1 to 6, characterized in that the molar ratio of (meth)acrylic anhydride to the diamine is between 1 and 1.2.

8. Process according to any one of Claims 1 to 7, characterized in that the quantity of polymerization inhibitor is such that the weight ratio of this inhibitor to the (meth)acrylic anhydride is greater than or equal to 0.25%.

**Patentansprüche**

1. Verfahren zur Synthese von N-Dialkylaminoalkyl(meth)acrylamid der Formel:

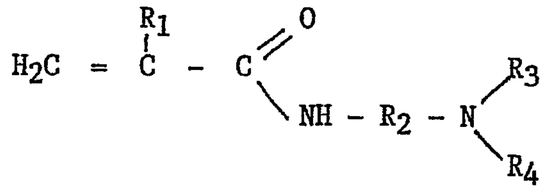

in der

– $R_1$ ein Methylradikal oder ein Wasserstoffatom ist,

– $R_2$ eine lineare oder verzweigte Alkylgruppe mit höchstens 10 Kohlenstoffatomen ist, und

– $R_3$ und $R_4$, die identisch oder verschieden sind, sein können:

. entweder zwei aliphatische Alkylgruppen mit 1 bis 4 Kohlenstoffatomen,

. oder $R_3$ eine aliphatische Alkylgruppe mit 1 bis 4 Kohlenstoffatomen und $R_4$ eine cycloaliphatische Gruppe mit 5 bis 6 Kohlenstoffatomen,

. oder $R_3$ und $R_4$ zwei cycloaliphatische Gruppen mit 5 bis 6 Kohlenstoffatomen,

. oder $R_3$ und $R_4$ mit dem Stickstoffatom, mit dem sie chemisch verbunden sind, eine heterocycloaliphatische Gruppe bilden,

durch Reaktion ausgehend von einem (Meth)acrylsäureanhydrid der Formel:

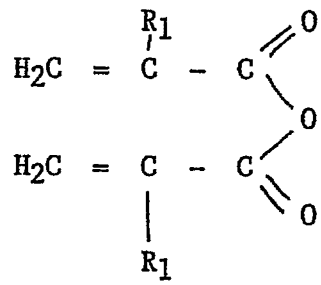

in Gegenwart mindestens eines Polymerisationsinhibitors, dadurch gekennzeichnet, daß man das genannte Anhydrid mit einem Diamin der Formel:

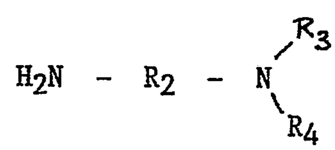

bei einer Temperatur zwischen 70 und 100°C und in Gegenwart von Sauerstoff reagieren läßt.

2. Verfahren nach Anspruch 1, dadurch gekennzeichnet, daß das erhaltene N-Dialkylaminoalkyl(meth)acrylamid durch Destillation abtrennt und daß man dazu dem Reaktionsmilieu vor und/oder nach der Reaktion zwischen dem (Meth)acrylsäureanhydrid und dem Diamin ein gegenüber dem Reaktionsmilieu inertes Lösungsmittel zugibt, das mit dem Reaktionsmilieu ein homogenes Milieu bildet und eine Siedetemperatur besitzt, die zwischen jener der (Meth)acrylsäure und jener des N-Dialkylaminoalkyl(meth)acrylats liegt.

3. Verfahren nach Anspruch 2, dadurch gekennzeichnet, daß die Menge des in das Reaktionsmilieu eingebrachten Lösungsmittels zwischen 10 und 50

Gew.-% bezogen auf die gesamte Charge der Reaktionsteilnehmer beträgt.

4. Verfahren nach Anspruch 3, dadurch gekennzeichnet, daß die in das Reaktionsmilieu eingebrachte Menge zwischen 20 und 30 Gew.-% bezogen auf die Gesamtcharge der Reaktionsteilnehmer beträgt.

5. Verfahren nach irgendeinem der Ansprüche 2 bis 4, dadurch gekennzeichnet, daß das inerte Lösungsmittel Trichlor-1,2,4-benzol oder eines seiner Isomere ist.

6. Verfahren nach Anspruch 1, dadurch gekennzeichnet, daß das erhaltene N-Dialkylaminoalkyl(meth)acrylat durch Extrahieren mit einem organischen Lösungsmittel nach Neutralisierung des Reaktionsmilieus mit einer starken Base abgetrennt wird.

7. Verfahren nach irgendeinem der Ansprüche 1 bis 6, dadurch gekennzeichnet, daß das Molverhältnis des (Meth)acrylsäureanhydrids zum Diamin zwischen 1 und 1,2 beträgt.

8. Verfahren nach irgendeinem der Ansprüche 1 bis 7, dadurch gekennzeichnet, daß die Menge des Polymerisationsinhibitors so ist, daß das Gewichtsverhältnis dieses Inhibitors zum (Meth)-acrylsäureanhydrid mehr als oder gleich 0,25% beträgt.